(19) Europäisches Patentamt
European Patent Office
Office européen des brevets

(11) Numéro de publication: **0 074 873**
**B1**

(12) **FASCICULE DE BREVET EUROPEEN**

(45) Date de publication du fascicule du brevet: **21.11.84**

(51) Int. Cl.³: **C 07 C 93/06**, A 61 K 31/13

(21) Numéro de dépôt: **82401583.8**

(22) Date de dépôt: **25.08.82**

(54) **Dérivés de phénoxy-3 propanol-2, leur préparation et leur application en thérapeutique.**

(30) Priorité: **10.09.81 FR 8117137**

(43) Date de publication de la demande:
**23.03.83 Bulletin 83/12**

(45) Mention de la délivrance du brevet:
**21.11.84 Bulletin 84/47**

(84) Etats contractants désignés:
**AT BE CH DE FR GB IT LI LU NL SE**

(56) Documents cités:
**FR - A - 2 330 383**
**FR - A - 2 367 053**

**Le dossier contient des informations techniques présentées postérieurement au dépôt de la demande et ne figurant pas dans le présent fascicule.**

(73) Titulaire: **SYNTHELABO, 58, rue de la Glacière, F-75621 Paris Cedex 13 (FR)**

(72) Inventeur: **Wick, Alexander, 10 Boulevard des Plants, F-78860 Saint Nom La Breteche (FR)**
Inventeur: **Binet, Jean, 12 Hameau de la Gondole, F-91650 Breuillet (FR)**

(74) Mandataire: **Ludwig, Jacques et al, SYNTHELABO Service Brevets 58, rue de la Glacière, F-75621 Paris Cedex 13 (FR)**

**EP 0 074 873 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne des dérivés de phénoxy-3 propanol-2, leur préparation et leur application en thérapeutique.

Les composés de l'invention répondent à la formule (I)

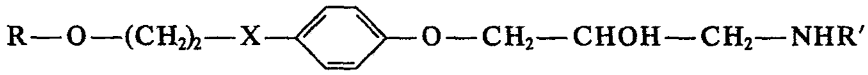

(I)

dans laquelle

R représente un radical cycloalkyle de 5 ou 6 atomes de carbone,
X représente un atome d'oxygène ou une liaison, et
R' représente le radical isopropyle ou tertiobutyle.

Des composés ayant une structure chimique et des applications analogues à celles des composés de l'invention sont décrits dans les FR-A-2 330 383 et FR-A-2 367 053.

Les composés de l'invention possèdent un carbone asymétrique; les racémates et les énantiomères (I) font partie de l'invention.

Selon l'invention on peut préparer les composés selon l'un des 3 schémas suivants:

Schéma 1 — X = O

On part du tosylate de cycloalcoxyéthyle (II)

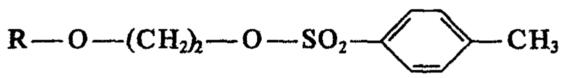

(II)

que l'on fait réagir avec le benzyloxy-4 phénol pour obtenir le cycloalcoxyéthoxy-1 benzyloxy-4 benzène (III)

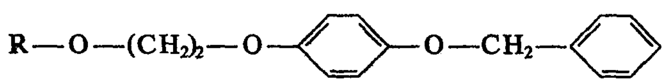

(III)

que l'on débenzyle; on fait réagir le phénol correspondant de formule (IV)

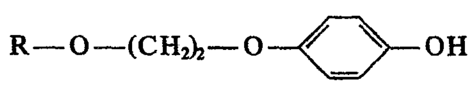

(IV)

avec l'épichlorhydrine et enfin on fait réagir le composé obtenu de formule (V)

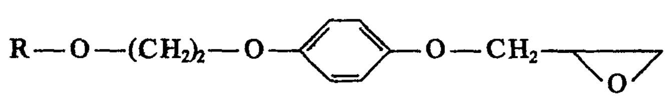

(V)

avec l'amine $R'NH_2$ pour obtenir le composé (I, X = O).

Schéma 2 — X = liaison

On fait réagir le tosylate de (benzyloxy-4 phényl)-2 éthyle

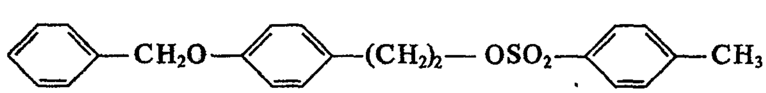

(VI)

avec le cycloalcanoate de sodium de formule (VII)

R—O—Na (VII)

on débenzyle ensuite le composé obtenu (VIII)

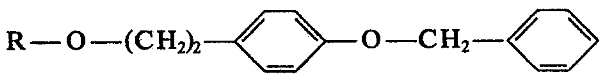

(VIII)

en phénol correspondant que l'on fait réagir avec l'épichlorhydrine pour obtenir le composé (IX)

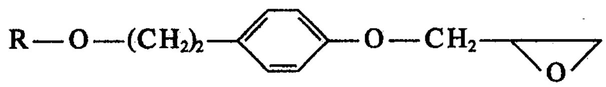

(IX)

que l'on fait réagir avec l'amine $R'NH_2$ pour obtenir le composé (I, X = liaison).

Schéma 3 – X = liaison

On fait réagir une diazocétone de formule (X)

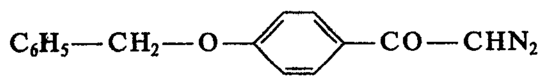

(X)

avec le cycloalcanol ROH (XI) puis on réduit complètement et débenzyle le composé obtenu de formule (XII)

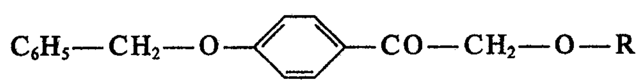

(XII)

en composé

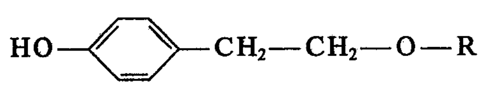

(XIII)

que l'on fait réagir avec l'épichlorhydrine pour donner le composé (XIV)

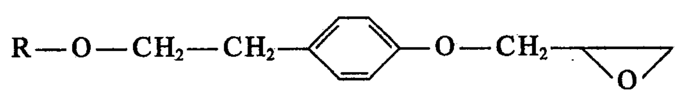

(XIV)

qui est condensé avec l'amine $R'NH_2$ pour obtenir le composé (I, X = liaison).

Les énantiomères des composés (I) peuvent être obtenus soit par séparation à l'aide d'un acide optiquement actif, soit et préférablement par réaction directe entre le sel de sodium d'un phénol

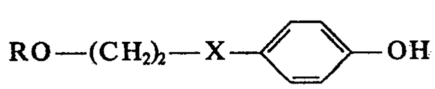

et l'énantiomère souhaité de l'oxazolidine de formule (XV)

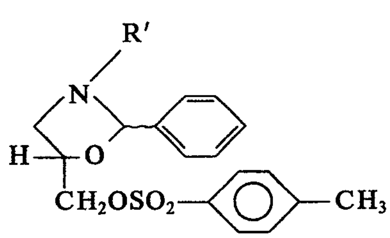

(XV)

en solution dans un solvant aprotique tel que le diméthylformamide à une température de 30 à 80°C,

suivi par une hydrolyse en milieu acide.

Les oxazolidines optiquement actives de formule (XV) sont décrites dans la littérature, par exemple par J. J. BALDWIN et al., J. Med. Chem. 20, 1024, (1977).

Les exemples suivants illustrent l'invention.

Les analyses et les spectres IR et RMN ont confirmé la structure des composés.

## Exemple 1

### (Cyclopentyloxy-2 éthoxy)-4 phénoxy-3 isopropylamino-1 propanol-2 et son malonate.

#### 1. Cyclopentyloxy-2 éthanol

On introduit à 0°C, dans un autoclave de 500 ml, 200 g (2,32 mole) de cyclopentanol, 38,5 $cm^3$ (0,77 mole) d'oxyde d'éthylène et 2,3 g (0,1 atome-g) de sodium métallique. On chauffe ensuite le mélange pendant 4 heures à 75°C. On laisse refroidir l'autoclave, puis on distille le mélange réactionnel. On obtient le composé bouillant à 90—92°C sous une pression de 15 mm.

#### 2. Tosylate de cyclopentyloxy-2 éthyle

On refroidit à —5°C, un mélange de 150 $cm^3$ de pyridine et 38 g de chlorure de tosyle. A cette température, on ajoute, goutte à goutte, en agitant, 24 g (0,185 mole) de cyclopentyloxy-2 éthanol dans 100 $cm^3$ de pyridine. On laisse ensuite remonter lentement la température et on laisse reposer le mélange réactionnel une nuit. Puis on le verse sur un mélange d'eau glacée et de 260 $cm^3$ d'HCl concentré et on extrait avec de l'éther. La phase éthérée est lavée à l'eau, séchée et évaporée à sec. Le tosylate obtenu est utilisé tel quel pour l'étape suivante.

#### 3. Ether benzylique du (cyclopentyloxy-2 éthoxy)-4 phénol

On ajoute, goutte à goutte, 24 g (0,12 mole) de benzyloxy-4 phénol en solution dans 100 $cm^3$ de DMF, à 6,2 g (0,132 mole) de NaH à 50% en suspension dans 50 $cm^3$ de DMF.

Le dégagement gazeux terminé, on ajoute alors 36 g (0,127 mole) de tosylate de cyclopentyloxy-2 éthyle en solution dans 100 $cm^3$ de DMF. On agite une demi heure. Puis on chauffe 2 heures à 55/60°C en agitant. On verse le mélange réactionnel sur de l'eau glacée. On extrait à l'éther. La phase éthérée est lavée avec de la soude diluée, puis à l'eau, séchée et évaporée à sec.

#### 4. (Cyclopentyloxy-2 éthoxy)-4 phénol

On débenzyle à 45°C, sous 50 psi d'$H_2$, 36 g (0,115 mole) de l'éther précédent dans 300 $cm^3$ de méthanol, en présence de Pd sur charbon à 5%. La réaction terminée, on filtre le catalyseur et évapore le filtrat à sec. On récupère une huile brute que l'on purifie par passage sur une colonne de silice en éluant au chloroforme.

#### 5. [(Cyclopentyloxy-2 éthoxy)-4 phénoxy]-3 époxy-1,2 propane

On agite 20 g (0,09 mole) de (cyclopentyloxy-2 éthoxy)-4 phénol avec 90 $cm^3$ de soude 1N jusqu'à obtention d'une solution limpide, puis on ajoute 12,5 g (0,135 mole) d'épichlorhydrine et on agite le mélange 24 heures, à la température ambiante. On extrait ensuite le mélange réactionnel à l'éther. La phase éthérée est lavée à la soude diluée, puis à l'eau, séchée sur $MgSO_4$, puis évaporée à sec.

#### 6. Malonate de [(cyclopentyloxy-2 éthoxy)-4 phénoxy]-3 isopropylamino-1 propanol-2

On porte à la température du reflux pendant 20 heures, 20 g de l'époxyde brut obtenu précédemment dans 150 $cm^3$ d'isopropylamine.

Puis on évapore à sec, on dissout le résidu à l'acide chlorhydrique dilué et extrait à l'éther 2 fois. La phase aqueuse est alcalinisée avec de la soude. On extrait la base au chlorure de méthylène. La phase organique est lavée à l'eau, séchée et évaporée à sec. La base huileuse est transformée en malonate neutre fondant à 96—98°C après recristallisation dans un mélange éthanol-éther.

Exemple 2

[(Cyclopentyloxy-2 éthyl)-4 phénoxy]-3 isopropylamino-1 propanol-2

1. Ether benzylique du (cyclopentyloxy-2 éthyl)-4 phénol

Dans un ballon de 250 ml on introduit 24,11 g de cyclopentanol puis 1,63 g de sodium. On chauffe à 110—120 °C jusqu'à disparition du sodium. On refroidit le mélange et on ajoute 80 ml de benzène. On introduit alors, par fractions, 26,77 g du tosylate de (benzyloxy-4 phényl)-2 éthanol. On chauffe à 80°C pendant 3 heures. On chasse le benzène, reprend par de l'eau et extrait avec de l'acétate d'éthyle. On obtient une huile.

2. (Cyclopentyloxy-2 éthyl)-4 phénol

On débenzyle 13,54 g du composé obtenu précédemment, en solution dans 250 ml de méthanol, en présence de charbon palladié à 5%, sous une pression de 55 psi d'hydrogène. Après filtration du catalyseur, et évaporation du filtrat, on obtient le produit.

3. [(Cyclopentyloxy-2 éthyl)-4 phénoxy]-3 époxy-1,2 propane

Dans un ballon de 500 ml, on dissout 8,95 g du phénol obtenu sous 2 dans 80 $cm^3$ de soude. On introduit 17,85 g (0,193 mole) d'épichlorhydrine, et agite le mélange réactionnel pendant 12 heures à la température ambiante. Après extraction à l'éther, lavage à l'eau et séchage sur sulfate de magnésium on obtient le composé cherché.

4. [(Cyclopentyloxy-2 éthyl)-4 phénoxy]-3 isopropylamino-1 propanol-2

Dans un ballon de 250 ml on chauffe à la température de reflux pendant 11 heures 12,8 g de l'époxyde otenu précédemment avec 50 ml d'isopropylamine. On élimine l'excès d'amine et reprend le mélange par de l'eau, on acicifie avec HCl dilué et extrait avec de l'éther. La phase aqueuse est alcalinisée avec NaOH et extraite avec de l'éther. On lave la phase organique avec de l'eau, sèche sur $MgSO_4$ filtre, évapore. On obtient après recristallisation dans l'héxane le produit cherché sous forme de base fondant à 66°C.

Exemple 3

[(Cyclohexyloxy-2 éthyl)-4 phénoxy]-3 isopropylamino-1 propanol-2
et son fumarate neutre

1. Cyclohexyloxyméthyl (benzyloxy-4 phényl) méthanone

Dans un ballon d'un litre, on introduit 100 g (1 mole) de cyclohexanol et 33 g (0,13 mole) de benzyloxy-4 diazo-2 acétophénone (J. JEFFREYS, J. Chem. Soc. (1956), 4451). On chauffe le mélange au bain d'eau à 35°C sous agitation magnétique. On introduit en deux fois 4 $cm^3$ d'éthérate de trifluorure de bore. On laisse la solution 4 heures au bain d'eau, puis on chasse le cyclohexane sous pression réduite. On purifie le produit par chromatographie sur silice en éluant par un mélange cyclohexane/acétate d'éthyle 7/3. On fait recristalliser le produit dans du cyclohexane.

2. (Cyclohexyloxy-2 éthyl)-4 phénol

On hydrogène sous une pression d'hydrogène de 50 psi, à une température de 35 à 40°C, 16 g (0,049 mole) de cyclohéxyloxyméthyl-(benzyloxy-4 phényl)-méthanone en solution dans 150 $cm^3$ de méthanol contenant quelques gouttes de $HClO_4$ à 70% et 1 g de Pd/C à 5%. On filtre le catalyseur, évapore le solvant sous vide et chromatographie l'huile résiduelle sur une colonne de silice (éluant cyclohexane/acétate d'éthyle 8/2). On recueille une huile d'indice de réfraction

$n_D^{18} = 1,5335$;  $Eb_{0,01} = 130°C$.

3. [(Cyclohexyloxy-2 éthyl)-4 phénoxy]-3 époxy-1,2 propane

Dans un ballon on introduit 13,3 g (0,06 mole) de [cyclohexyloxy-2 éthyl]-4 phénol, 3,04 g (0,076 mole) de soude en pastilles et 50 $cm^3$ d'eau. On agite à la température ambiante et on introduit, quand la solution est devenue homogène, 18,6 g (16 $cm^3$, 0,2 mole) d'épichlorhydrine. On maintient l'agitation à la température ambiante pendant 16 heures. On extrait à l'éther et on lave la phase organique à l'eau. On sèche sur sulfate de magnésium et chasse l'éther. On obtient une huile que l'on utilise directement dans l'étape suivante.

4. Fumarate neutre de [(cyclohexyloxy-2 éthyl)-4 phénoxy]-3 isopropylamino-1 propanol-2

Dans un ballon de 200 $cm^3$, on introduit 5,6 g (0,019 mole) de [(cyclohexyloxy-2 éthyl)-4 phénoxy]-3 époxy-1,2 propane, 40 $cm^3$ de méthanol et 11,73 g (17 $cm^3$, 0,198 mole) d'isopropylamine. On chauffe au bain d'huile tout en agitant magnétiquement à 50°C, durant 7 heures. On chasse le méthanol sous pression réduite. On reprend l'huile dans 40 $cm^3$ d'HCl 3N, et on extrait à l'éther. On alcalinise la phase aqueuse et extrait à l'éther. La pâte est triturée dans du pentane et de l'éther de pétrole, on récupère une huile.

On introduit dans 30 $cm^3$ d'acétone, 3,65 g (0,01 mole) de [(cyclohexyloxy-2 éthyl)-4 phénoxy]-3 isopropylamino-1 propanol-2. On ajoute à cette solution 1,16 g (0,01 mole) d'acide fumarique dans 20 $cm^3$ d'acétone; on filtre la solution finale sur papier et il y a, après une nuit de repos, précipitation de cristaux floconneux blancs. On essore et lave avec 30 $cm^3$ d'acétone. Le sel obtenu est recristallisé dans un minimum d'acétone.

F = 100°C.

Exemple 4

Isomère (S)(−) du [(cyclopentyloxy-2 éthyl)-4 phénoxy]-3 isopropylamino-1 propanol-2

On ajoute 7,4 g (0,036 mole) de (cyclopentyloxy-2 éthyl)-4 phénol en solution dans 10 $cm^3$ de DMF à une suspension de 1,8 g (0,036 mole) de NaH à 50% (lavé préalablement avec du toluène) dans 10 $cm^3$ de DMF.

Lorsque la sodation est terminée, on ajoute 13,6 g (0,036 mole) du tosylate de (S) isopropyl-1 phényl-2 hydroxyméthyl-5 oxazolidine en solution dans 10 ml de DMF, puis on chauffe le mélange à 50—60°C. On maintient le chauffage pendant 5 heures, refroidit le mélange puis le verse sur de l'eau et extrait avec de l'éther. On lave la phase éthérée avec de l'eau, sèche, filtre et évapore. On reprend l'huile résiduelle dans de l'eau, acidifie avec 15 $cm^3$ d'acide chlorhydrique concentré, agite 1/2 heure puis extrait avec de l'éther. On alcalinise la phase aqueuse avec de la soude concentrée (20 $cm^3$) et extrait avec de l'éther. On lave la phase éthérée avec de l'eau, la sèche sur $MgSO_4$, filtre et évapore. On obtient une huile à partir de laquelle on prépare le chlorhydrate de la manière suivante:

On solubilise la base huileuse dans un minimum d'acétone, élimine un insoluble par filtration, acidifie avec de l'éther chlorhydrique, puis on ajoute de l'éther jusqu'à obtention d'un trouble léger. Le produit cristallise. On le filtre, le sèche et le fait recristalliser dans un mélange acétone/éther.

F = 97—100°C.
$[\alpha]_D = -19{,}64°$ (c = 0,011 ; $CH_3OH$)

La base purifiée obtenue par alcalinisation du chlorhydrate cristallise et fond à 40—41°C.

Tableau

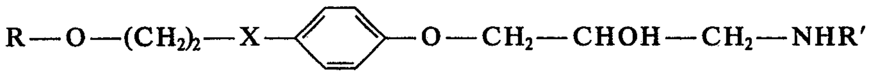

| Composé | R | R' | X | Forme | F (°C) |
|---|---|---|---|---|---|
| 1 | 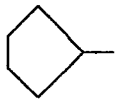 | 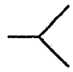 | 0 | malonate | 96–98 |
| 2 | 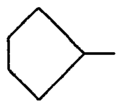 | 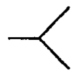 | liaison | base | 66 |
| 3 (S) | 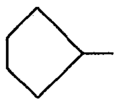 | 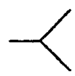 | liaison | chlorhydrate | 97–100 |
| 4 | 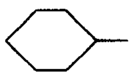 | 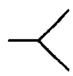 | 0 | chlorhydrate | 112 |
| 5 | 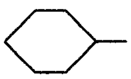 | 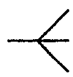 | 0 | chlorhydrate | 138–139 |
| 6 | 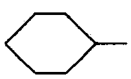 | 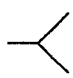 | liaison | fumarate | 100 |

Les composés (I) de l'invention ont été soumis à une série d'essais pharmacologiques qui ont mis en évidence leurs propriétés intéressantes dans le domaine cardio-vasculaire.

La toxicité aigüe par voies orale et intraveineuse a été évaluée chez des souris mâles CDI, d'un poids moyen de 20 g. On a noté la mortalité sur une période de 5 jours suivant l'administration des composés. La dose létale 50% (DL 50) a été calculée selon Litchfield et Wilcoxon (J. Pharmacol. Exp. Ther 1944, 95, 99). La DL 50 va de 600 à 2000 mg/kg per os.

Etudes sur organes isolès

On a utilisé des oreillettes isolées prélevées sur des rats pesant 250 à 350 g, maintenues dans la solution de Moran (en g/l: $NaCl = 7{,}02$; KCl: 0,42; $CaCl_2 = 0{,}24$; $MgCl_2 = 0{,}20$; $NaHCO_3 = 2{,}0$; glucose = 1,8), oxygénée (95% $O_2$ – 5% $CO_2$) à la température de 31 °C. On a étudié la tachycardie et l'augmentation de la force contractile provoquée par l'isoprénaline (courbe dose – réponse à l'agoniste) avant et après l'addition de l'antagoniste (composé (I) ou substance de référence) et on a calculé le $PA_2$ de chacun d'eux par la méthode de Arunlakshana et Schild (Brit. J. Pharmacol. 1959, 14, 48) le $pA_2$ représentant le logarithme de la concentration molaire d'antagoniste compétitif nécessitant une dose d'agoniste deux fois plus forte pour provoquer les mêmes réponses que celles obtenues en l'absence d'antagoniste. Le $pA_2$ des composés est compris entre 8 et 10,5.

Tous les composés (I) possèdent une activité inhibitrice vis-à-vis des effets cardiaques de l'isoprénaline mais non vis-à-vis des effets hypotenseurs de cette dernière: ce sont donc bien des agents de blocage sélectif des récepteurs $\beta_1$-adrénergiques, c'est-à-dire les récepteurs $\beta$-adrénergiques situés au niveau du coeur et non vis-à-vis des récepteurs $\beta_2$ adrénergiques situés au niveau des vaisseaux.

Les résultats qui précèdent montrent que les composés de l'invention sont utilisables en médecine humaine et vétérinaire, dans les maladies cardiovasculaires et, notamment, dans les affections coronariennes, les affections atteignant le myocarde et les troubles du rythme cardiaque.

De plus, les composés abaissent de manière significative la pression intraoculaire induite chez des lapins hypertendus. Les composés de l'invention peuvent donc aussi être utilisés pour le traitement du glaucome.

L'invention comprend, par conséquent, toutes compositions pharmaceutiques renfermant les composés de formule générale (I) et leurs sels comme principes actifs en association avec tous excipients appropriés à leur administration par voie orale, ou rectale, ou parentérale. Ces compositions pharmaceutiques peuvent également contenir d'autres substances médicamenteuses avec lesquelles ces composés et leurs sels sont pharmaceutiquement et thérapeutiquement compatibles.

Pour l'administration paar voie orale, on utilise toutes les formes pharmaceutiques appropriées à cette voie, c'est-à-dire les comprimés, dragées, gélules, capsules, cachets, solutions et suspensions buvables, la prise unitaire du principe actif pouvant varier entre 5 et 200 mg et la dose quotidienne étant comprise entre 10 et 500 mg.

Pour l'administration par voie endorectale, on utilise des suppositoires renfermant 2 à 150 mg de principe actif et administrés au malade à raison de 1 à 3 par 24 heures.

Pour l'administration par voie parentérale, on utilise des solutés injectables stabilisés et tamponnés, préparés à l'avance ou extemporanément. La dose de principe actif par prise unitaire peut varier entre 1 et 10 mg et la dose quotidienne est comprise entre 3 et 50 mg.

**Revendications pour les états contractants: BE, CH, DE, FR, GB, IT, LI, LU, NL, SE**

1. Dérivés de phénoxy-3 propanol-2, sous la forme de racémates ou d'énantiomères, répondant à la formule (I)

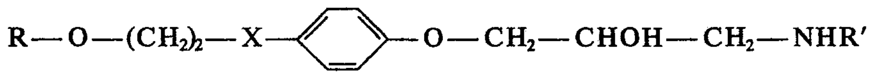

(I)

dans laquelle

R représente un radical cycloalkyle de 5 ou 6 atomes de carbone,
X représente un atome d'oxygène ou une liaison, et
R' représente le radical isopropyle ou tertiobutyle.

2. Le (cyclopentyloxy-2 éthyl)-4 phénoxy-3 isopropylamino-1 propanol-2.
3. L'isomère (S) (−) du (cyclopentyloxy-2 éthyl)-4 phénoxy-3 isopropylamino-1 propanol-2.
4. Le (cyclopentyloxy-2 éthoxy)-4 phénoxy-3 isopropylamino-1 propanol-2 et son malonate.
5. Procédé de préparation des composés selon la revendication 1, procédé caractérisé par le fait que

— lorsque X est un atome d'oxygène, on fait réagir le tosylate de cycloalcoxyéthyle (II)

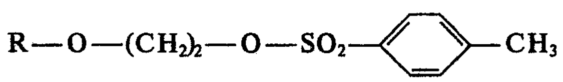

(II)

avec le benzyloxy-4 phénol pour obtenir le cycloalcoxyéthoxy-1 benzyloxy-4 benzène (III)

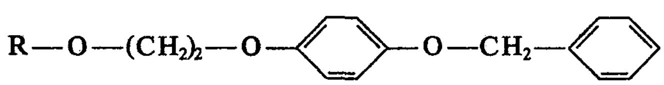

(III)

que l'on débenzyle; on fait réagir le phénol correspondant de formule (IV)

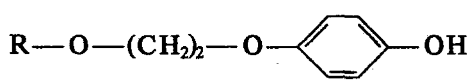

(IV)

avec l'épichlorhydrine et enfin on fait réagir le composé obtenu de formule (V)

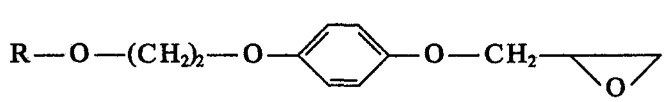

(V)

avec l'amine $R'NH_2$ pour obtenir le composé (I, X = O).

— lorsque X est une liaison

- soit on fait réagir le tosylate de (benzyloxy-4 phényl)-2 éthyle

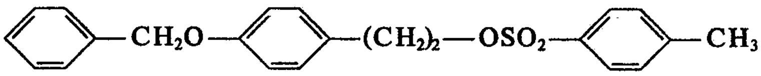 (VI)

avec le cycloalcanoate de sodium de formule (VII)

$R—O—Na$ (VII)

on débenzyle ensuite le composé obtenu (VIII)

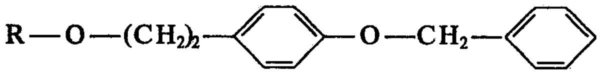 (VIII)

en phénol correspondant que l'on fait réagir avec l'épichlorhydrine pour obtenir le composé (IX)

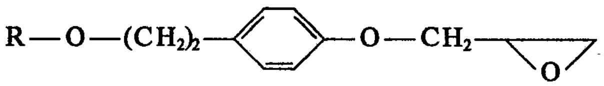 (IX)

que l'on fait réagir avec l'amine $R'NH_2$ pour obtenir le composé (I, X = liaison).

- soit on fait réagir une diazocétone de formule (X)

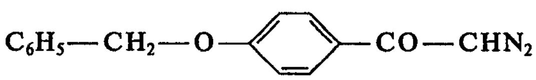 (X)

avec le cycloalcanol ROH (XI) puis on réduit complétement et débenzyle le composé obtenu de formule (XII)

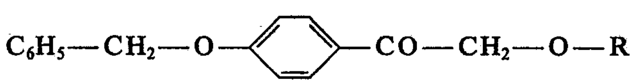 (XII)

en composé

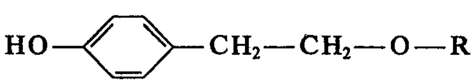 (XIII)

que l'on fait réagir avec l'epichlorhydrine pour donner le composé (XIV)

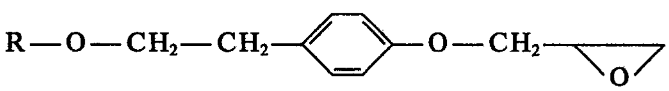 (XIV)

qui est condensé avec l'amine $R'NH_2$ pour obtenir le composé (I, X = liaison).

6. Procédé de préparation des énantiomères des composés selon la revendication 1, procédé caractérisé en ce qu'on fait réagir le sel de sodium d'un phénol

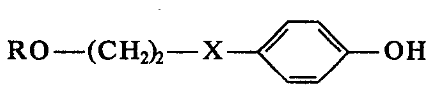

et l'énantiomère souhaité de l'oxazolidine de formule (XV)

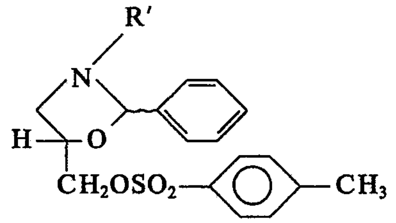

(XV)

en solution dans un solvant tel que le diméthylformamide à une température de 30 à 80 °C, suivi par une hydrolyse en milieu acide.

7. Médicament caractérisé en ce qu'il contient un composé tel que spécifié dans l'une quelconque des revendications 1 à 4.

8. Composition pharmaceutique caractérisée en ce qu'elle contient un composé tel que spécifié dans l'une quelconque des revendications 1 à 4 en association avec tout excipient approprié.

**Revendications pour état contractant: AT**

1. Procédé de préparation de dérivés de phénoxy-3 propanol-2, sous la forme de racémates ou d'énantiomères, répondant à la formule (I)

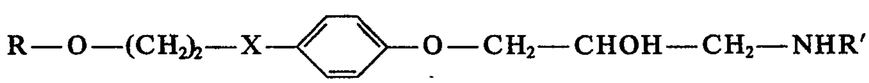

(I)

dans laquelle

R représente un radical cycloalkyle de 5 ou 6 atomes de carbone,
X représente un atome d'oxygène ou une liaison et
R' représente le radical isopropyle ou tertiobutyle,

procédé caractérisé par le fait que

— lorsque X est un atome d'oxygène, on fait réagir le tosylate de cycloalcoxyéthyle (II)

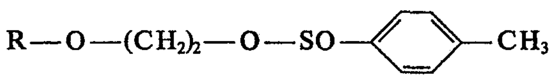

(II)

avec le benzyloxy-4 phénol pour obtenir le cycloalcoxyéthoxy-1 benzyloxy-4 benzène (III)

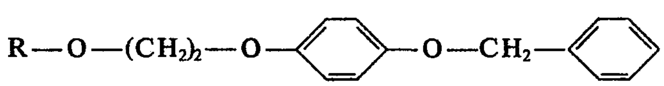

(III)

que l'on débenzyle; on fait réagir le phénol correspondant de formule (IV)

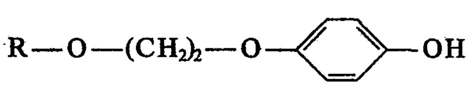

(IV)

avec l'épichlorhydrine et enfin on fait réagir le composé obtenu de formule (V)

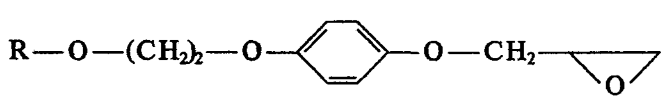

(V)

avec l'amine $R'NH_2$ pour obtenir le composé (I, X = O).

— lorsque X est une liaison

- soit on fait réagir le tosylate de (benzyloxy-4 phényl)-2 éthyle

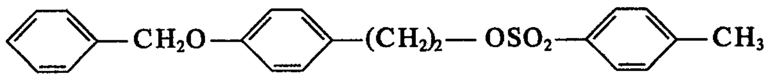 (VI)

avec le cycloalcanoate de sodium de formule (VII)

R—O—Na (VII)

on débenzyle ensuite le composé obtenu (VIII)

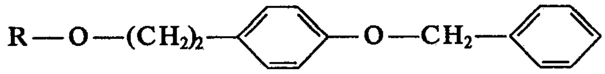 (VIII)

en phénol correspondant que l'on fait réagir avec l'épichlorhydrine pour obtenir le composé (IX)

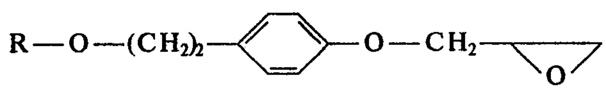 (IX)

que l'on fait réagir avec l'amine R'$NH_2$ pour obtenir le composé (I, X = liaison).

- soit on fait réagir une diazocétone de formule (X)

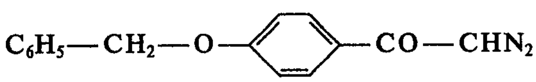 (X)

avec le cycloalcanol ROH (XI) puis on réduit complétement et débenzyle le composé obtenu de formule (XII)

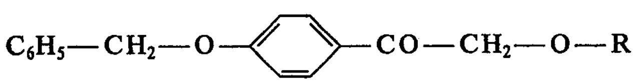 (XII)

en composé

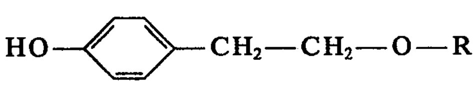 (XIII)

que l'on fait réagir avec l'epichlorhydrine pour donner le composé (XIV)

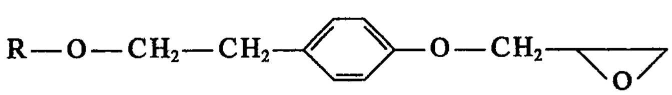 (XIV)

qui est condensé avec l'amine R'$NH_2$ pour obtenir le composé (I, X = liaison).

2. Procédé de préparation des énantiomères des composés de formule (I) définis dans la revendication 1, procédé caractérisé en ce qu'on fait réagir le sel de sodium d'un phénol

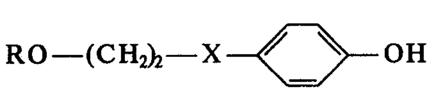

et l'énantiomère souhaité de l'oxazolidine de formule (XV)

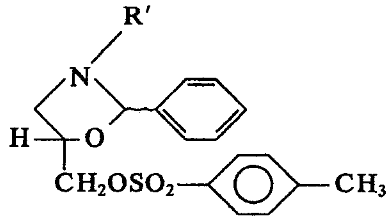

(XV)

en solution dans un solvant tel que le diméthylformamide à une température de 30 à 80°C, suivi par une hydrolyse en milieu acide.

**Patentansprüche für die Vertragsstaaten: BE, CH, DE, FR, GB, IT, LI, LU, NL, SE**

1. Derivate des 3-Phenoxy-2-propanols in Form der Racemate oder der Enantiomeren, die der Formel (I)

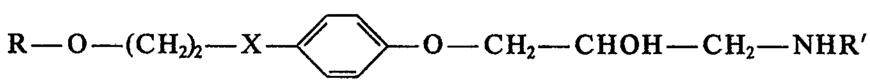

(I)

entsprechen, in welcher

R für einen Cycloalkylrest mit 5 oder 6 Kohlenstoffatomen,
X für ein Sauerstoffatom oder eine Bindung und
R' für den Isopropyl- oder tert.-Butylrest steht.

2. 4-(2-Cyclopentyloxyäthyl)-3-phenoxy-1-isopropylamino-2-propanol.
3. Das (S)(−)-Isomere von 4-(2-Cyclopentyloxyäthyl)-3-phenoxy-1-isopropylamino-2-propanol.
4. 4-(2-Cyclopentyloxyäthoxy)-3-phenoxy-1-isopropylamino-2-propanol und dessen Malonat.
5. Verfahren zur Herstellung der Verbindungen nach Anspruch 1, dadurch gekennzeichnet, daß

— wenn X für ein Sauerstoffatom steht, man das Cycloalkoxyäthyltosylat (II)

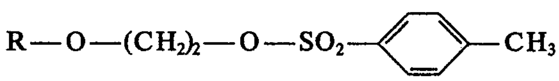

(II)

mit 4-Benzyloxyphenol zur Gewinnung des 1-Cycloalkoxyäthoxy-4-benzyloxybenzols (III)

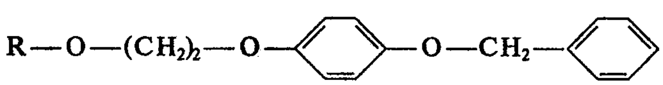

(III)

reagieren läßt, das man entbenzyliert; man läßt das entsprechende Phenol der Formel (IV)

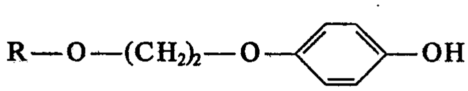

(IV)

mit Epichlorhydrin reagieren und setzt schließlich die erhaltene Verbindung der Formel (V)

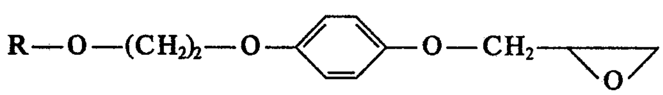

(V)

mit dem Amin $R'NH_2$ zur Gewinnung der Verbindung (I, X = 0) um,

— wenn X für eine Bindung steht,

- man entweder das 2-(4-Benzyloxyphenyl)äthyltosylat

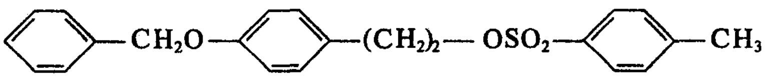
(VI)

mit dem Natriumzykloalkanoat der Formel (VII)

R—O—Na (VII)

umsetzt, anschließend die erhaltene Verbindung (VIII)

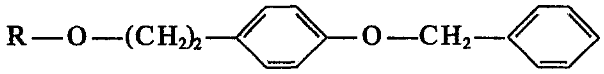
(VIII)

zum entsprechenden Phenol entbenzyliert, das man mit Epichlorhydrin zur Gewinnung der Verbindung (IX)

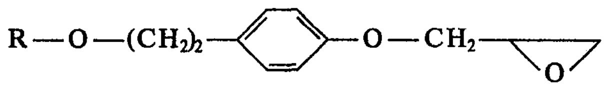
(IX)

umsetzt, welche man mit dem Amin $R'NH_2$ zur Gewinnung der Verbindung (I, X = Bindung) zur Reaktion bringt;

– oder daß man ein Diazoketon der Formel (X)

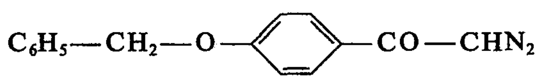
(X)

mit dem Cycloalkanol ROH (XI) umsetzt, dann vollständig reduziert und die erhaltene Verbindung (XII)

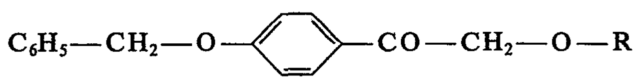
(XII)

zur Verbindung

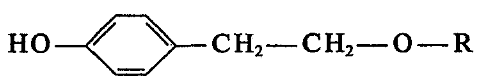
(XIII)

entbenzyliert und daß man diese mit Epichlorhydrin zur Gewinnung der Verbindung (XIV)

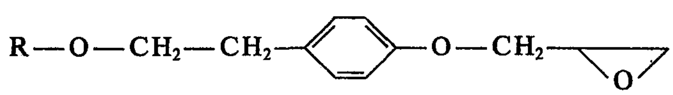
(XIV)

reagieren läßt, welche zur Gewinnung der Verbindung (I, X = Bindung) mit dem Amin $R'NH_2$ kondensiert wird.

6. Verfahren zur Herstellung der Enantiomeren der Verbindungen nach Anspruch 1, dadurch gekennzeichnet, daß man das Natriumsalz eines Phenols

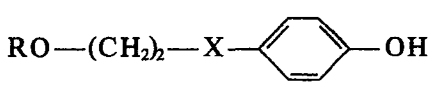

mit dem gewünschten Enantiomer des Oxazolidins der Formel (XV)

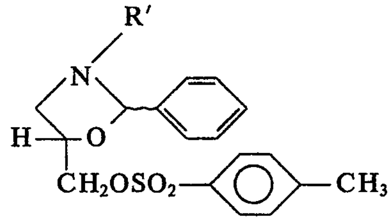

(XV)

in Lösung in einem Lösungsmittel wie Dimethylformamid bei einer Temperatur von 30 bis 80 °C reagieren und eine Hydrolyse im sauren Milieu folgen läßt.

7. Arzneimittel, dadurch gekennzeichnet, daß es eine Verbindung nach irgend einem der Ansprüche 1 bis 4 enthält.

8. Pharmazeutische Zusammensetzung, dadurch gekennzeichnet, daß sie eine in irgend einem der Ansprüche 1 bis 4 dargelegte Verbindung in Kombination mit irgend einem geeigneten Träger enthält.

**Patentansprüche für den Vertragsstaat: AT**

1. Verfahren zur Herstellung von Derivaten des 3-Phenoxy-2-propanols in Form der Racemate oder der Enantiomeren, die der Formel (I)

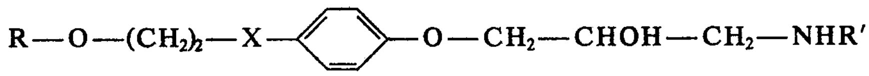

(I)

entsprechen, in welcher

R für einen Cycloalkylrest mit 5 oder 6 Kohlenstoffatomen,
X für ein Sauerstoffatom oder eine Bindung und
R' für den Isopropyl- oder tert.Butylrest stehen,

dadurch gekennzeichnet, daß

— wenn X für ein Sauerstoffatom steht, man das Cycloalkoxyäthyltosylat (II)

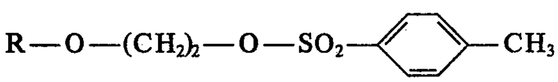

(II)

mit 4-Benzyloxyphenol zur Gewinnung des 1-Cycloalkoxyäthoxy-4-benzyloxybenzols (III)

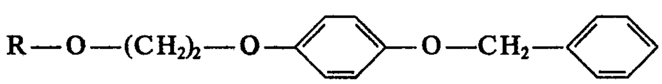

(III)

reagieren läßt, das man entbenzyliert; man läßt das entsprechende Phenol der Formel (IV)

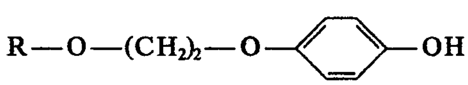

(IV)

mit Epichlorhydrin reagieren und setzt schließlich die erhaltene Verbindung der Formel (V)

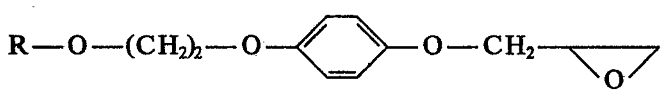

(V)

mit dem Amin $R'NH_2$ zur Gewinnung der Verbindung (I, X = O) um,

— wenn X für eine Bindung steht,

- man entweder das 2-(4-Benzyloxyphenyl)äthyltosylat

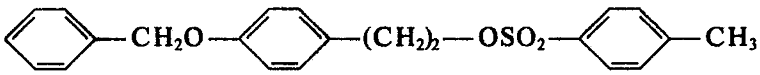

(VI)

mit dem Natriumzykloalkanoat der Formel (VII)

$R—O—Na$ (VII)

umsetzt, anschließend die erhaltene Verbindung (VIII)

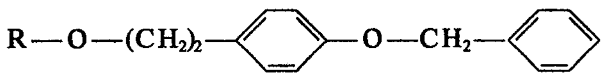

(VIII)

zum entsprechenden Phenol entbenzyliert, das man mit Epichlorhydrin zur Gewinnung der Verbindung (IX)

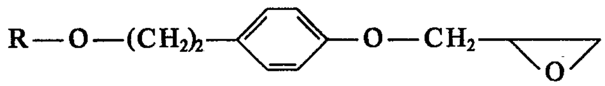

(IX)

umsetzt, welche man mit dem Amin $R'NH_2$ zur Gewinnung der Verbindung (I, X = Bindung) zur Reaktion bringt;

- oder daß man ein Diazoketon der Formel (X)

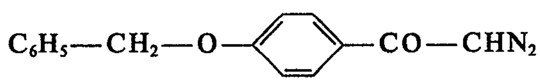

(X)

mit dem Cycloalkanol ROH (XI) umsetzt, dann vollständig reduziert und die erhaltene Verbindung (XII)

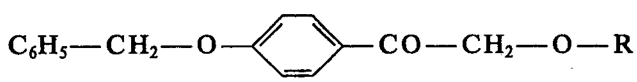

(XII)

zur Verbindung

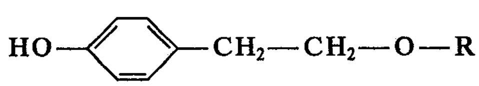

(XIII)

entbenzyliert und daß man diese mit Epichlorhydrin zur Gewinnung der Verbindung (XIV)

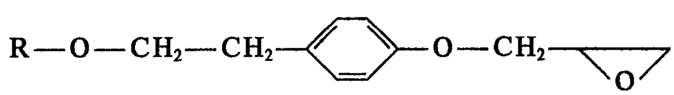

(XIV)

reagieren läßt, welche zur Gewinnung der Verbindung (I, X = Bindung) mit dem Amin $R'NH_2$ kondensiert wird.

2. Verfahren zur Herstellung von Enantiomeren von Verbindungen der in Anspruch 1 definierten Formel (I), dadurch gekennzeichnet, daß man das Natriumsalz eines Phenols

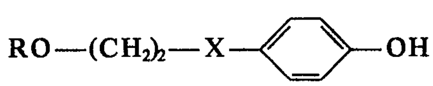

mit dem gewünschten Enantiomer des Oxazolidins der Formel (XV)

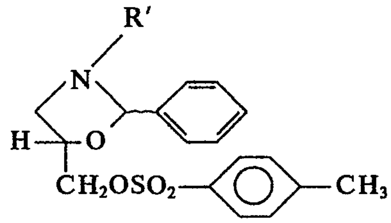

(XV)

in Lösung in einem Lösungsmittel wie Dimethylformamid bei einer Temperatur von 30 bis 800 reagieren und eine Hydrolyse im sauren Milieu folgen läßt.

**Claims for the contracting states: BE, CH, DE, FR, GB, IT, LI, LU, NL, SE**

1. 3-Phenoxypropan-2-ol derivatives, in the form of racemates or enantiomers, corresponding to formula (I).

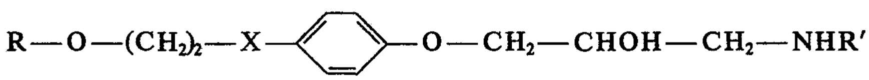

(I)

wherein

R represents a cycloalkyl group of 5 or 6 carbon atoms,
X represents an oxygen atom or a bond, and
R' represents the isopropyl or tertiobutyl radical.

2. 4-(2-cyclopentyloxy-ethyl)-3-phenoxy-1-isopropylamino-propan-2-ol.
3. The (S)(—)-isomer of 4-(2-cyclopentyloxy-ethyl)-3-phenoxy-1-isopropylamino-propan-2-ol.
4. 4-(2-cyclopentyloxy-ethoxy)-3-phenoxy-1-isopropylamino-propan-2-ol and the malonate thereof.
5. Process for preparing the compounds according to claim 1, characterized in that

— when X is an oxygen atom, a cycloalkoxyethyl tosylate (II)

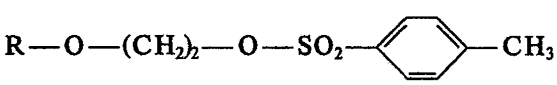

(II)

is reacted with 4-benzyloxy-phenol to produce 1-cycloalkoxyethoxy-4-benzyloxy-benzene (III)

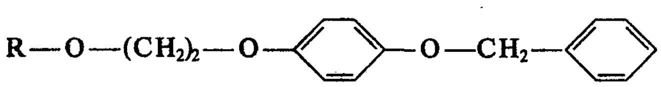

(III)

which becomes debenzylated; then the corresponding phenol of formula (IV)

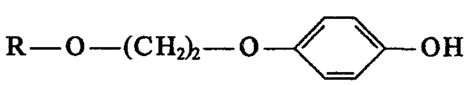

(IV)

is reacted with epichlorhydrin, and finally the compound obtained, of formula (V)

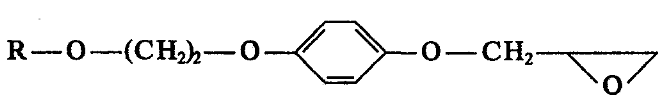

(V)

is reacted with the amine $R'NH_2$ to produce the compound (I, X = O)

— when X is a bond

- either the 2-(4-benzyloxy-phenyl)-ethyl tosylate

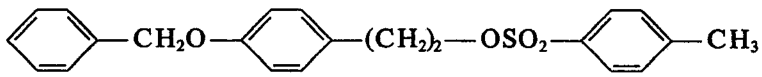 (VI)

is reacted with the sodium cycloalcanoate of formula (VII)

R—O—Na (VII)

then the compound obtained (VIII) is debenzylated

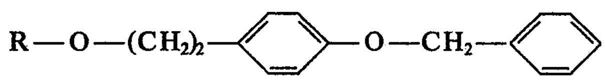 (VIII)

to produce the corresponding phenol which is reacted with epichlorhydrin to produce compound (IX)

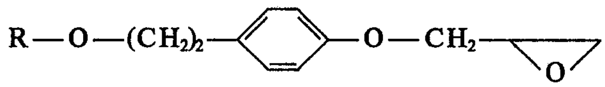 (IX)

which is reacted with the amine $R'NH_2$ to produce the compound (I, X = bond)

- either a diazoketone of formula (X)

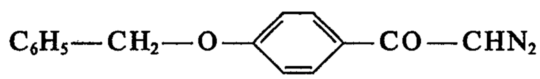 (X)

is reacted with the cycloalkanol ROH (XI) and then the compound obtained of formula (XII)

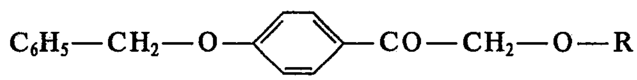 (XII)

is reduced completely and debenzylated to produce the compound

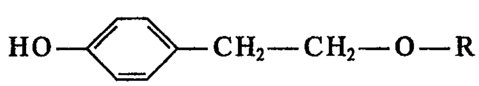 (XIII)

which is reacted with epichlorhydrin to produce the compound (XIV)

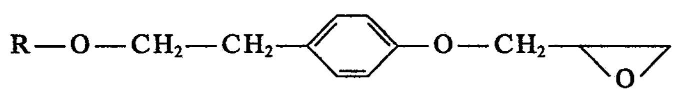 (XIV)

which becomes condensed with the amine $R'NH_2$ to produce the compound (I, X = bond).

6. Process for preparing enantiomers of the compounds according to claim 1, characterized in that the sodium salt of a phenol

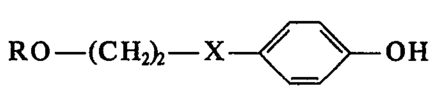

and the appropriate enantiomer of the oxazolidine of formula (XV)

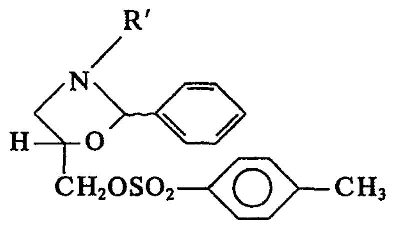

(XV)

are reacted in a solvent such as dimethylformamide at a temperature of 30 to 80°C, followed by a hydrolysis in acidic medium.

7. Drug characterized in that it contains a compound as specified in any one of claims 1 to 4.

8. A pharmaceutical composition characterized in that it contains a compound as specified in any one of claims 1 to 4 in combination with any appropriate excipient.

**Claims for the contracting state: AT**

1. Process for preparing 3-Phenoxypropan-2-ol derivatives, in the form of racemates or enantiomers, corresponding to formula (I)

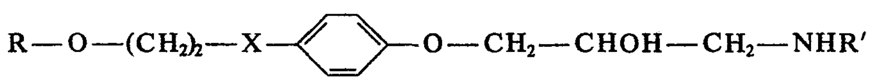

(I)

wherein

R represents a cycloalkyl group of 5 or 6 carbon atoms,
X represents an oxygen atom or a bond, and
R' represents the isopropyl or tertiobutyl radical,

process characterized in that

- when X is an oxygen atom, a cycloalkoxyethyl tosylate (II)

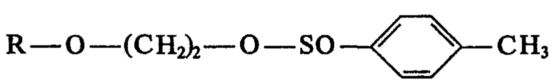

(II)

is reacted with 4-benzyloxy-phenol to produce 1-cycloalkoxyethoxy-4-benzyloxy-benzene (III)

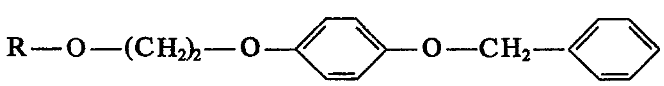

(III)

which becomes debenzylated; then the corresponding phenol of formula (IV)

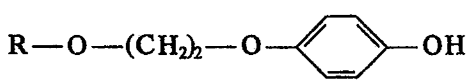

(IV)

is reacted with epichlorhydrin, and finally the compound obtained, of formula (V)

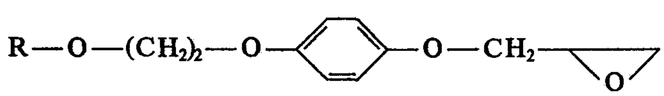

(V)

is reacted with the amine $R'NH_2$ to produce the compound (I, X = O)

- when X is a bond

- either the 2-(4-benzyloxy-phenyl)-ethyl tosylate

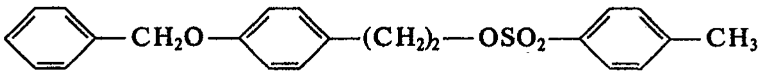

(VI)

is reacted with the sodium cycloalcanoate of formula (VII)

R—O—Na (VII)

then the compound obtained (VIII) is debenzylated

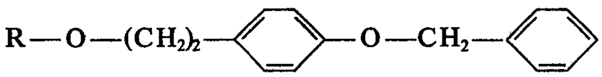

(VIII)

to produce the corresponding phenol which is reacted with epichlorhydrin to produce compound (IX)

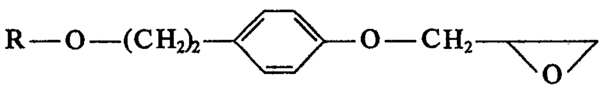

(IX)

which is reacted with the amine $R'NH_2$ to produce the compound (I, X = bond)

- either a diazoketone of formula (X)

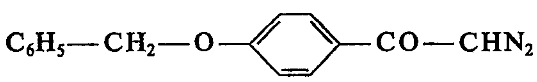

(X)

is reacted with the cycloalkanol ROH (XI) and then the compound obtained of formula (XII)

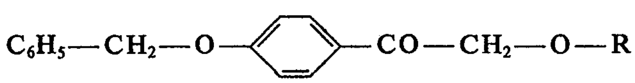

(XII)

is reduced completely and debenzylated to produce the compound

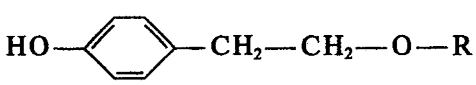

(XIII)

which is reacted with epichlorhydrin to produce the compound (XIV)

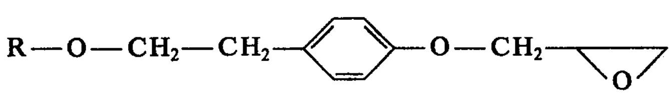

(XIV)

which becomes condensed with the amine $R'NH_2$ to produce the compound (I, X = bond).

2. Process for preparing the enantiomers of the compounds of formula (I) as specified in claim 1, process characterized in that the sodium salt of a phenol

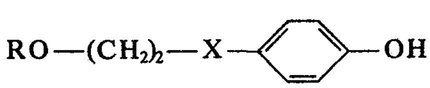

and the appropriate enantiomer of the oxazolidine of formula (XV)

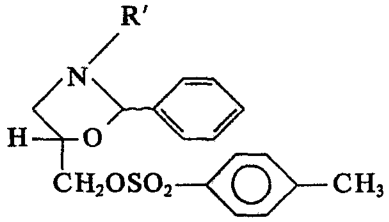

(XV)

are reacted in a solvent such as dimethylformamide at a temperature of 30 to 80°C, followed by a hydrolysis in acidic medium.